(19) Europäisches Patentamt
European Patent Office
Office européen des brevets

(11) Numéro de publication: **0 113 625**
**B1**

(12) # FASCICULE DE BREVET EUROPEEN

(45) Date de publication du fascicule du brevet: **10.07.85**

(51) Int. Cl.⁴: **C 07 C 17/32**, C 07 C 149/32, C 07 C 148/00, C 07 C 41/30

(21) Numéro de dépôt: **83402441.6**

(22) Date de dépôt: **16.12.83**

(54) **Procédé de préparation de composés benzéniques trifluorométhylés.**

(30) Priorité: **28.12.82 FR 8221887**

(43) Date de publication de la demande: **18.07.84 Bulletin 84/29**

(45) Mention de la délivrance du brevet: **10.07.85 Bulletin 85/28**

(84) Etats contractants désignés: **AT BE CH DE FR GB IT LI LU NL SE**

(56) Documents cités:
**EP - A - 0 008 453**
**US - A - 4 207 266**

(73) Titulaire: **RHONE-POULENC SPECIALITES CHIMIQUES, "Les Miroirs" 18, Avenue d'Alsace, F-92400 Courbevoie (FR)**

(72) Inventeur: **Desbois, Michel, 526, Chemin du Bois, F-69140 Rillieux (FR)**

(74) Mandataire: **Cazes, Jean-Marie et al, RHONE-POULENC RECHERCHES Service Brevets Chimie et Polymères 25, quai Paul Doumer, F-92408 Courbevoie Cedex (FR)**

EP 0 113 625 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention concerne un procédé de préparation de dérivés benzéniques trifluorométhylés. Elle concerne plus particulièrement un procédé de trifluorométhylation de dérivés benzéniques désactivés.

On connait dans l'art antérieur le brevet européen n° 08 453 qui concerne la préparation de tels dérivés par réaction sur un dérivé aromatique du tétrachlorure de carbone et d'acide fluorhydrique anhydre, à une température comprise entre 50 et 140° C.

Ce procédé appliqué à des produits désactivés du type dichloro ou trichlorobenzène, donne un taux de transformation et/ou un rendement nul ou extrêmement faible même à des températures élevées ou pour des durées de réaction prolongées.

Dans le brevet des Etats-Unis d'Amérique n° 4 207 266 est décrit un procédé de préparation de dérivés trifluorométhylés aromatiques selon lequel on fait réagir un composé aromatique de préférence désactivé, avec un tétrahalogénure de carbone et de l'hydrogène fluoré en présence d'un catalyseur constitué d'acide de Lewis ou de Bronsted fort.

Ces acides forts sont choisis parmi les fluorures et chlorures des éléments des groupes 4B à 6B et 3A à 5A de la classification périodique.

Parmi ces acides sont choisis tout particulièrement le trichlorure d'antimoine, le trifluorure d'antimoine, le pentachlorure d'antimoine, le pentafluorure d'antimoine, le tétrachlorure de titane, le tétrafluorure de titane, le pentachlorure de niobium, le pentafluorure de niobium, le pentafluorure de tantale.

Les rendements molaires de réaction en dérivés trifluorométhylés sont très faibles et atteignent au maximum 32% dans l'exemple 3 où l'on met en présence le dichlorobenzène et un catalyseur à base de tétrachlorure de titane.

Ces rendements extrêment faibles sont dus à la présence d'une grande quantité de benzophénones résultant d'une réaction secondaire parasitaire entre le produit formé c'est-à-dire le dichlorotrifluorométhylbenzène et le produit initial c'est-à-dire le dichlorobenzène.

Il ressort bien de l'art antérieur qu'il existe un besoin actuel de réaliser une réaction de trifluorméthylation notamment sur des dérivés di ou trihalogénoaromatiques, avec des rendements, des températures et des durées de réaction compatibles avec une exploitation industrielle.

La présente demande a pour but de révéler le moyen d'atteindre ces objectifs.

L'invention concerne un procédé de préparation de composés benzéniques trifluorométhylés directement sur le noyau par réaction sur un dérivé benzénique, de tétrahalogénure de carbone et d'acide fluorhydrique en présence d'un catalyseur acide de Lewis, caractérisé en ce que le dérivé benzénique est un di ou trihalogénobenzène, les halogènes pouvant être remplacés en totalité ou en partie par des groupes perhalogénoalcoxy ou perhalogénoalkylthio et l'acide de Lewis est du trifluorure de bore utilisé à une pression absolue d'au moins 1 bar.

Il est connu de l'homme de l'art que les groupes halogénoalcoxy ou halogénoalkylthio ont une force électroattractrice de la même nature et à peu près de la même intensité que les halogènes.

Ainsi peuvent être considérés comme équivalents au sens de la présente invention tous les substituants qui ont une force électroattractrice ayant les mêmes caractéristiques que celle des halogènes.

Selon un mode de réalisation particulier du procédé selon l'invention, le dérivé benzénique répond à la formule:

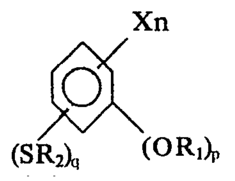

(I)

où:

- X est un halogène
- $R_1$ et $R_2$ sont des groupes perhalogénoalkyle, $R_1$ et $R_2$ étant identiques ou différents et ayant chacun de 1 à 3 atomes de carbone
- n, p et q sont compris chacun entre 0 et 3
- et la somme $n + p + q$ est égale à 2 ou 3

L'intérêt du procédé est tout particulièrement marqué, car les composés qu'il permet alors d'obtenir sont très utiles au plan industriel, quand on met en oeuvre:

- un composé de formule (I) dans laquelle $p = 0$
- ou un composé de formule (I) dans laquelle $q = 0$

Cet intérêt est encore plus marqué quand dans la formule (I) p=0 et q=0 car les composés trifluorométhylé di ou trihalogénés sont particulièrement recherchés au plan industriel.

L'invention a consisté à trouver de façon surprenante que le trifluorure de bore, non spécifiquement décrit dans le brevet des Etats-Unis d'Amérique n° 4 207 266, donnait sur les dérivés benzéniques désactivés de la présente invention une réaction avec des rendements inattendus en produit recherché et peu de produits indésirables tels que les benzophénones.

Le trifluorure de bore présente en outre de grands aventages par rapport aux catalyseurs utilisés dans le brevet des Etats-Unis d'Amérique n° 4 207 266. C'est un produit industriel, donc disponible sur le marché, et il se présente sous une forme gazeuse ce qui permet de le récupérer facilement et de le recycler en fin de réaction.

Les catalyseurs utilisés dans le brevet des Etats-Unis d'Amérique n° 4 207 266 tels que le pentachlorure d'antimoine sont solides. De ce fait, ils ne sont récupérables et recyclables sans opération accessoire que lorsque les produits issus de la réaction sont gazeux ce qui n'est pas le cas dans la présente invention.

L'emploi de trifluorure de bore comme catalyseur est déjà connu dans l'art antérieur dans une réaction de O-trifluorométhylation d'un paranitrophénol. (Brevet des Etats-Unis d'Amérique n° 4 157 344 exemple 19.) Cette trifluorométhylation est réalisée directement sur l'oxygène du phénol alors que le problème ici envisagé est une trifluorométhylation sur le carbone du cycle benzénique. Il est bien connu par l'homme du métier que la réaction de substitution électrophile sur l'oxygène d'un phénol ou d'un dérivé phénolique est beaucoup plus facile à réaliser que la substitution électrophile sur le carbone du cycle benzénique, car elle ne dépend que très peu de la désactivation du noyau.

On peut citer comme exemples de produits pouvant être mis en oeuvre selon la présente invention:

les dichlorobenzènes,
les dibromobenzènes,
les difluorobenzènes,
les diiodobenzènes,
les trichlorobenzènes,
les tribromobenzènes,
les trifluorobenzènes,
les triiodobenzènes,
les dichlorofluorobenzènes
les difluorochlorobenzènes,
les dibromofluorobenzènes,
les chlorofluorobromobenzènes,
les chlorofluorobenzènes,
les bromofluorobenzènes,
les chlorobromobenzènes,
les chlorotrifluorométhoxybenzènes,
les bis-trifluorométhoxybenzènes,
les fluorotrichlorométhoxybenzènes,
les chlorotrifluorométhylthiobenzènes,
les fluorotribromométhylthiobenzènes,
les dichlorotrifluoromethoxybenzènes,
les chlorofluorotrifluorométhylthiobenzènes.

Le tétrahalogénure de carbone mis en oeuvre peut contenir notamment des halogènes choisis parmi le fluor, le chlore ou le brome.

On peut citer comme exemples de tétrahalogénure de carbone pouvant être mis en oeuvre selon la présente invention: $CCl_4$, $CBr_4$, $CBrCl_3$, $CFCl_3$, $CF_3Cl$.

L'halogénure préféré selon la présente invention est le tétrachlorure de carbone du fait de sa disponibilité sur le marché et de son coût nettement inférieur à celui des autres halogénures.

D'autre part, à la température de la réaction, il est liquide et sert de solvant au meme titre que l'acide fluorhydrique ce qui n'est pas le cas de tous les autres halogénures.

L'acide fluorhydrique mis en oeuvre est de préférence anhydre.

La mise en oeuvre d'acide fluorhydrique aqueux entraînerait une consommation inutile de trifluorure de bore sous forme HF, $BF_3$, $H_2O$ ($H_3O^+BF_4^-$).

D'autre part, la présence d'eau peut provoquer l'hydrolyse au moins partielle du groupe trifluorométhyle en groupe carboxylique ce qui entrainera une perte de rendement en produit désiré.

Des quantités stoechiométriques d'une mole de tétrahalogénure de carbone et de trois moles d'acide fluorhydrique sont nécessaires pour que la réaction ait lieu.

Toutefois, il est, d'une manière générale, préférable d'utiliser des quantités de tétrahalogénure de carbone et d'acide fluorhydrique nettement supérieures. Ainsi, il est particulièrement intéressant de les utiliser tous les deux comme solvants de la réaction.

Du point de vue industriel, il est économiquement préférable d'utiliser une quantité d'acide fluorhydrique telle que le rapport molaire de l'acide fluorhydrique au dérivé benzénique de départ soit compris entre 5 et 100. Encore plus préférentiellement, ce rapport est compris entre 20 et 60.

Il est aussi économiquement préférable d'utiliser une quantité de tétrahalogénure de carbone telle que le rapport molaire du tétrahalogénure de carbone au dérivé benzénique de départ soit compris entre 1 et 5 et encore plus préférentiellement entre 1 et 3.

On préfère plus particulièrement utiliser une quantité de trifluorure de bore telle que la pression absolue de $BF_3$ dans l'enceinte réactionnelle soit comprise entre 2 et 20 bars. Une pression supérieure è 20 bars n'est pas exclue de l'invention mais n'apporte pas d'avantages particuliers.

Le procédé de l'invention est de préférence mis en oeuvre à une température comprise entre 20° C et 150° C.

La durée de la réaction varie avec les matières premières mises en jeu (c'est-à-dire avec la désactivation du noyau aromatique et avec le tétrahalogénure de carbone) ainsi qu'avec la température de la réaction.

Les produits obtenus selon le procédé de l'invention ont pour formule générale:

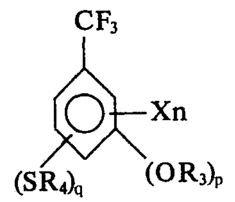

dans laquelle:

- X, n, p, q ont la signification précédente:
- $R_3$ et $R_4$ représentent:
  - le groupement $CF_3$ si $R_1$ et $R_2$ sont un groupe perhalométhyle
  - un groupement perhalogénoéthyle ou perhalogénopropyle dont le carbone en $\alpha$ de l'oxygène ou du soufre porte deux atomes de fluor, les autres atomes de carbone portant les mêmes atomes d'halogène que dans $R_1$ et $R_2$.

En effet lors de la réaction qui s'effectue en milieu HF, les groupements tel que $OCCl_3$, $OCBr_3$, $OCF_2$, $OCCl_2F$, $OCF_2Cl$ etc... ainsi que leurs équivalents se transforment en $OCF_3$ et $SCF_3$ par échange des atomes d'halogène avec des atomes de fluor du milieu.

Par contre quand le radical $R_1$ ou $R_2$ contient deux ou trois atomes de carbone, cette réaction d'échange des atomes d'halogène ne se fera que sur le carbone en $\alpha$ de l'hétéroatome, les autres atomes d'halogènes portés par les autres atomes de carbone des groupements $R_1$ et $R_2$ ne s'échangeant pas.

Ainsi, lors de la réaction les groupements $OCCl_2$, $CCl_3$, $OCBr_2$ $CCl_3$, $OCBrClCCl_3$, $OCCl_2$ $CCl_2$ $CCl_3$, $OCBr_2$ $CCl_2$ $CCl_3$ ainsi que leurs équivalents se transforment en $OCF_2$ $CCl_3$, $OCF_2$ $CCl_3$, $OCF_2$ $CCl_3$, $OCF_2$ $CCl_2$ $CCl_3$, $OCF_2$ $CCl_2$ $CCl_3$.

Les atomes d'halogènes fixés directement sur le noyau benzénique ne sont pas échangés dans les conditions de la réaction.

La position du groupement $CF_3$ obéit aux règles classiques de substitution.

Les dérivés trifluorométhylés obtenus selon la présente invention sont utilisés comme intermédiaires de synthèse dans l'industrie pharmaceutique et phytosanitaire.

On peut citer comme produits obtenus à l'aide du présent procédé:

les dichlorotrifluorométhylbenzènes,
les trichlorotrifluorométhylbenzènes,
les trifluorotrifluorométhylbenzènes,
les bromochlorofluorotrifluorométhylbenzènes,
les chlorotrifluorométhoxytrifluorométhylbenzènes,
les chlorofluorotrifluorométhylbenzènes,
les bromofluorotrifluorométhylbenzènes,
les dibromotrifluorométhylbenzènes,
les difluorotrifluorométhylbenzènes,
les diiodotrifluorométhylbenzènes,
les chlorobromotrifluorométhylbenzènes,
les bis-trifluorométhoxytrifluorométhylbenzènes,
les fluorotrifluorométhoxytrifluorométhylbenzènes,
les chlorotrifluorométhylthiotrifluorométhylbenzènes,
les fluorotrifluorométhylthiotrifluorométhylbenzènes,
les dichlorotrifluorométhoxytrifluorométhylbenzènes,
les chlorofluorotrifluorométhylthiotrifluorométhylbenzènes...

Les exemples suivants sont donnés à titre indicatif et ne peuvent être considérés comme une limite du domaine et de l'esprit de l'invention.

## Exemple 1

Dans un réacteur inox de 250 ml agité par barreau aimanté et maintenu aux environs de 10° C par un bain d'eau glacée, on introduit 100 g (5 m) de HF anhydre, 14,7 g (0,1 m) d'orthodichlorobenzène et 30,8 g (0,2 m) de tétrachlorure de carbone. Le réacteur est fermé puis on introduit du trifluorure de bore gazeux jusqu'à la pression de 6 bars ce qui correspond à une quantité de 0,15 m dissoute à la température de l'essai. On chauffe à 80° C pendant 3 heures sous bonne agitation.

La pression passe d'environ 16 bars à environ 30 bars. Le réacteur est alors refroidi vers 10° C puis amené à la pression atmosphérique. Le mélange réactionnel ainsi obtenu est coulé sur environ 200 g de glace pilée puis extrait par 3 fois 50 $cm^3$ de chlorure de méthylène. Cette phase organique est lavée par 3 fois 100 $cm^3$ d'eau, séchée puis évaporée partiellement. Les analyses par chromatographie en phase gazeuse et spectrométrie IR indiquent la présence de 0,063 m du mélange des dichloro-2,3 et dichloro-3,4 trifluorométhylbenzène et de 0,034 m de produits du type benzophénones soit un rendement de 63% pour un taux de transformation de 100%.

## Exemple 2

On opère de la même façon qu'à l'exemple 1 mais sans introduction de $BF_3$. On récupère intégralement l'orthodichlorobenzène de départ. Aucune trace de dichloro-2,3 ou dichloro-3,4-trifluorméthyl benzène n'a pu être mise en évidence.

## Exemple 3

On opère de la même façon qu'à l'exemple 2 mais en travaillant à 140° C pendant 5 heures. Après traitements et analyses on observe un rendement de 30% pour un taux de transformation de 33% (soit un rendement réel d'environ 10%).

## Exemple 4

On opère de la même façon qu'à l'exemple 1 avec les composés et conditions suivants:

| | |
|---|---|
| p-dichlorobenzène | 14,7 g (0,1 m) |
| tétrachlorure de carbone | 30,8 g (0,2 m) |
| Hf | 100 g (5 m) |
| $BF_3$ | 6 bars à 20° C (0,15 m) |
| température | 80° C |
| durée | 3 heures 30 |

Après traitements et analyses, on observe un rendement de 89% en dichloro-2,5 trifluorométhylbenzène pour un taux de transformation de 72,4% (soit un rendement réel d'environ (64%).

## Exemple 5

On opère de la même façon qu'à l'exemple 1 avec les composés et conditions suivants:

| | |
|---|---|
| m-dichlorobenzène | 14,7 g (0,1 m) |
| tétrachlorure de carbone | 46,2 g (0,3 m) |
| HF | 40 g (2 m) |
| $BF_3$ | 8 bars à 20° C (0,06 m) |
| température | 80° C |
| durée | 4 heures |

Après traitements et analyses, on observe un rendement de 70% en dichloro-2,4 trifluorométhylbenzène pour un taux de transformation de 100%.

Exemple 6

On opère de la même façon qu'à l'exemple 1 avec les composés et conditions suivants:

| | |
|---|---|
| trichloro-1,2,4 benzène | 18,2 g (0,1 m) |
| tétrachlorure de carbone | 30,8 g (0,2 m) |
| HF | 100 g (5 m) |
| $BF_3$ | 6 bars à 20° C (0,14 m) |
| température | 100° C |
| durée | 6 heures |

Après traitement et analyses, on observe un rendement de 95% en trichloro-2,4,5 trifluorométhylbenzène pour un taux de transformation de 25%.

Exemple 7

On opère de la même façon qu'à l'exemple 6 mais sons introduction de $BF_3$ et en travaillant à 140° C. On récupère intégralement le trichloro-1,2,4 benzène de départ. Aucune trace de trichloro, trifluorométhylbenzène n'a pu être mise en évidence.

Exemple 8

On opère de la même façon qu'à l'exemple 1 avec les composés et conditions suivants:

| | |
|---|---|
| p-chlorotrifluorométhoxybenzène | 19,7 g—(0,1 m) |
| tétrachlorure de carbone | 61,6 g—(0,4 m) |
| HF | 120 g (6 m) |
| $BF_3$ | 7 bars à 20° C (0,2 m) |
| température | 80° C |
| durée | 18 H 30 |

Après traitements et analyses, on observe un rendement de 90% en trifluorométhoxy-2 chloro-5 trifluorométhylbenzène et chloro-2 trifluorométhoxy-5 trifluorométhylbenzène pour un taux de transformation de 69%.

Exemple 9

On opère de la même façon qu'à l'exemple 1 avec les composés et conditions suivants:

| | |
|---|---|
| m-chlorofluorobenzène | 6,5 g (0,05 m) |
| tétrachlorure de carbone | 30,8 g (0,2 m) |
| HF | 50 g (2,5 m) |
| $BF_3$ | 6 bars à 20° C (0,075 m) |
| température | 86° C |
| durée | 4 H 40 |

Après traitements et analyses, on observe un rendement de 76% pour un taux de transformation de 100% en chloro-2 fluoro-4 trifluorométhylbenzène et fluoro-2 chloro-4 trifluorométhylbenzène.

Exemple 10

On opère de la même façon qu'à l'exemple 1 avec les composés et conditions suivants:

| | |
|---|---|
| o-bromofluorobenzène | 17,5 g (0,1 m) |
| tétrachlorure de carbone | 46,2 g (0,3 m) |
| HF | 60 g (3 m) |
| $BF_3$ | 6 bars à 20° C (0,09 m) |
| température | 90° C |
| durée | 16 H 30 |

Après traitements et analyses, on observe un rendement de 75% pour un taux de transformation de 65% en bromofluorotrifluorométhylbenzènes.

Exemple 11

On opère de la même façon qu'à l'exemple 1 avec les composés et conditions suivants:

| | |
|---|---|
| p-bistrichlorométhoxybenzène | 17,25 g (0,05 m) |
| Tétrachlorure de carbone | 31 g (0,2 m) |
| HF | 50 g (2,5 m) |
| $BF_3$ | 6 bars à 20° C (0,075 m) |
| Température | 120° C |
| Durée | 4 heures |

Après traitements et analyses, on observe un rendement de 10% en trifluorométhyl p-bistrifluorométhoxybenzène.

Exemple 12

On opère de la même façon qu'à l'exemple 1 avec les composés et conditions suivants:

| | |
|---|---|
| p-chlorotrichlorométhoxybenzène | 12,3 g (0,05 m) |
| Tétrachlorure de carbone | 31 g (0,2 m) |
| HF | 50 g (2,5 m) |
| $BF_3$ | 6 bars à 20° C (0,075 m) |
| Température | 120° C |
| Durée | 4 heures |

Après traitements et analyses, on observe un rendement de 43% en trifluorométhyl p-chlorotrifluorométhoxybenzène.

Exemple 13

On opère de la même façon qu'à l'exemple 1 avec les composés et conditions suivants:

| | |
|---|---|
| Dichloro-2,6 trichlorométhoxybenzène | 14 g (0,05 m) |
| Tétrachlorure de carbone | 31 g (0,2 m) |
| HF | 50 g (2,5 m) |
| $BF_3$ | 6 bars à 20° C (0,075 m) |
| Température | 122° C |
| Durée | 4 H 45 |

Après traitements et analyses, on observe un rendement de 17% en trifluorométhyldichloro-2,6 trifluorométhoxybenzène.

**Revendications**

1. Procédé de préparation de composés benzéniques trifluorométhylés directement sur le noyau aromatique par réaction sur un dérivé benzénique de tétrahalogénure de carbone, d'acide fluorhydrique et d'un catalyseur acide de Lewis, caractérisé en ce que le dérivé benzénique est un di ou trihalogénobenzène, les halogènes pouvant être remplacés en totalité ou en partie par des groupes per halogénoalcoxy ou perhalogénothioalkyle et l'acide de Lewis est du trifluorure de bore utilisé à une pression absolue d'au moins 1 bar.

2. Procédé selon la revendication 1 caractérisé en ce que le dérivé benzénique répond à la formule:

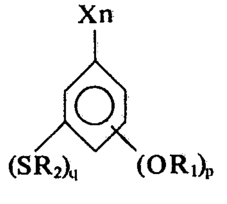

(I)

où:

- X est un halogène
- $R_1$ et $R_2$ sont des groupes perhalogénoalkyle, $R_1$ et $R_2$ étant identiques ou différents et ayant chacun de 1 à 3 atomes de carbone.
- n, p et q sont compris chacun entre 0 et 3 et la somme $n+p+q$ étant égale à 2 ou 3

3. Procédé selon la revendication 2 caractérisé en ce que dans la formule (I) $q=0$.
4. Procédé selon la revendication 2 caractérisé en ce que dans la formule (I) $p=0$.
5. Procédé selon la revendication 2 caractérisé en ce que dans la formule (I) $p=0$ et $q=0$.
6. Procédé selon l'une quelconque des revendications précédentes caractérisé en ce que l'on utilise une quantité de tétrahalogénure de carbone telle que le rapport molaire du tétrahalogénure de carbone au dérivé benzénique est compris entre 1 et 5.
7. Procédé selon l'un quelconque des revendications précédentes caractérisé en ce que le tétrahalogénure de carbone est $CCl_4$.
8. Procédé selon l'une quelconque des revendications précédentes caractérisé en ce que l'acide fluorhydrique mis en oeuvre est de l'acide fluorhydrique anhydre.
9. Procédé selon la revendication 8 caractérisé en ce que l'on utilise une quantité d'acide fluorhydrique telle que le rapport molaire de l'acide fluorhydrique au dérivé benzénique est compris entre 5 et 100.
10. Procédé de préparation selon la revendication 1 caractérisé en ce qu'on utilise une quantité de trifluorure de bore telle que la pression absolue de $BF_3$ dans l'enceinte réactionnelle soit comprise entre 2 et 20 bars.
11. Procédé selon l'une quelconque des revendications précédentes caractérisé en ce que la température de réaction est comprise entre 20 et 150.

**Patentansprüche**

1. Verfahren zur Herstellung von Benzolderivaten mit einer Trifluormethylgruppe direkt am aromatischen Ring durch Reaktion von Tetrahalogenkohlenstoff, Fluorwasserstoffsäure und einem Lewis-Säure-Katalysator mit einem Benzolderivat, dadurch gekennzeichnet, daß das Benzolderivat ein Di- oder Trihalogenbenzol ist, wobei die Halogenatome vollständig oder teilweise durch Perhalogenalkoxy- oder Perhalogenthioalkylgruppen ersetzt sein können, und dadurch, daß die Lewis-Säure Bortrifluorid ist, das bei einem absoluten Druck von mindestens 1 bar eingesetzt wird.
2. Verfahren nach Anspruch 1, dadurch gekennzeichnet, daß das Benzolderivat die Formel besitzt:

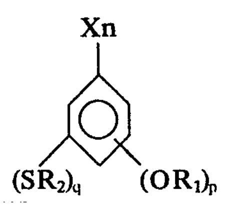

(I)

in der

- X ein Halogenatom ist,
- $R_1$ und $R_2$ Perhalogenalkylgruppen sind, wobei $R_1$ und $R_2$ gleich oder verschieden sein können und jeweils 1 bis 3 Kohlenstoffatome besitzen,
- n, p und q jeweils zwischen 0 und 3 liegen und die Summe $n+p+q$ gleich 2 oder 3 ist.

3. Verfahren nach Anspruch 2, dadurch gekennzeichnet, daß in der Formel I $q=0$ ist.
4. Verfahren nach Anspruch 2, dadurch gekennzeichnet, daß in der Formel I $p=0$ ist.
5. Verfahren nach Anspruch 2, dadurch gekennzeichnet, daß in der Formel I $p=0$ und $q=0$ ist.
6. Verfahren nach einem der vorangehenden Ansprüche, dadurch gekennzeichnet, daß man eine derartige Menge Tetrahalogenkohlenstoff einsetzt, daß das Molverhältnis zwischen Tetrahalogenkohlenstoff und Benzolderivat zwischen 1 und 5 liegt.
7. Verfahren nach einem der vorangehenden Ansprüche, dadurch gekennzeichnet, daß der Tetrahalogenkohlenstoff $CCl_4$ ist.
8. Verfahren nach einem der vorangehenden Ansprüche, dadurch gekennzeichnet, daß die eingesetzte Fluorwasserstoffsäure wasserfreie Fluorwasserstoffsäure ist.
9. Verfahren nach Anspruch 8, dadurch gekennzeichnet, daß man eine derartige Menge Fluorwasserstoffsäure einsetzt, daß das Molverhältnis von Fluorwasserstoffsäure zu Benzolderivat zwischen 5 und 100 liegt.
10. Verfahren nach Anspruch 1, dadurch gekennzeichnet, daß man eine derartige Menge Bortrifluorid einsetzt, daß der absolute Druck von $BF_3$ im Reaktionsraum zwischen 2 und 20 bar liegt.

11. Verfahren nach einem der vorangehenden Ansprüche, dadurch gekennzeichnet, daß die Reaktionstemperatur zwischen 20 und 150° C liegt.

## Claims

1. Process for the preparation of benzene compounds which are trifluoromethylated directly on the aromatic nucleus, by reaction of a carbon tetrahalide, hydrofluoric acid and a Lewis acid catalyst with a benzene derivative, characterised in that the benzene derivative is a dihalogenobenzene or trihalogenobenzene, the halogens being entirely or partially replaceable by halogenoalkoxy or perhalogenothioalkyl groups, and in that the Lewis acid is boron trifluoride used at an absolute pressure of at least 1 bar.

2. Process according to Claim 1, characterised in that the benzene derivative corresponds to the formula:

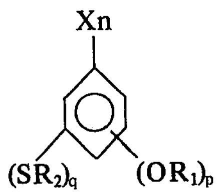

(I)

where:

- X is a halogen,
- $R_1$ and $R_2$ are perhalogenoalkyl groups, $R_1$ and $R_2$ being identical or different and each having 1 to 3 carbon atoms,
- n, p and q are each between 0 and 3 and the sum of $n+p+q$ is 2 or 3.

3. Process according to Claim 2, characterised in that in formula (I) $q=0$.

4. Process according to Claim 2, characterised in that in formula (I) $p=0$.

5. Process according to Claim 2, characterised in that in formula (I) $p=0$ and $q=0$.

6. Process according to any one of the preceding claims, characterised in that the amount of carbon tetrahalide used is such that the molar ratio of the carbon tetrahalide to the benzene derivative is between 1 and 5.

7. Process according to any one of the preceding claims, characterised in that the carbon tetrahalide is $CCl_4$.

8. Process according to any one of the preceding claims, characterised in that the hydrofluoric acid employed is anhydrous hydrofluoric acid.

9. Process according to Claim 8, characterised in that the amount of hydrofluoric acid used is such that the molar ratio of hydrofluoric acid to benzene derivative is between 5 and 100.

10. Process of preparation according to Claim 1, characterised in that the amount of boron trifluoride used is such that the absolute pressure of $BF_3$ in the reaction chamber is between 2 and 20 bar.

11. Process according to any one of the preceding claims, characterised in that the reaction temperature is between 20 and 150.